(19) **Europäisches Patentamt**
**European Patent Office**
**Office européen des brevets**

(11) **EP 1 689 222 B1**

(12) **EUROPEAN PATENT SPECIFICATION**

(45) Date of publication and mention
of the grant of the patent:
**03.03.2010 Bulletin 2010/09**

(51) Int Cl.:
***A01H 5/10*** (2006.01)   ***A23D 9/00*** (2006.01)

(21) Application number: **03782359.8**

(22) Date of filing: **08.12.2003**

(86) International application number:
**PCT/EP2003/014026**

(87) International publication number:
**WO 2005/046315 (26.05.2005 Gazette 2005/21)**

(54) **SUNFLOWER OIL, SEEDS AND PLANTS WITH MODIFIED FATTY ACID DISTRIBUTION IN THE TRIACYLGLYCEROL MOLECULE**

SONNENBLUMENÖL, SONNENBLUMENSAMEN UND PFLANZEN MIT MODIFIZIERTER FETTSÄUREVERTEILUNG IM TRIACYLGLYCEROLMOLEKÜL

HUILE, GRAINES ET PLANTES DE TOURNESOL AVEC DISTRIBUTION MODIFIEE DES ACIDES GRAS DANS LA MOLECULE DE TRIACYLGLYCEROL

(84) Designated Contracting States:
**AT BE BG CH CY CZ DE DK EE ES FI FR GB GR HU IE IT LI LU MC NL PT RO SE SI SK TR**

(30) Priority: **14.11.2003 PCT/EP03/13030**

(43) Date of publication of application:
**16.08.2006 Bulletin 2006/33**

(60) Divisional application:
**09160127.8**

(73) Proprietor: **Consejo Superior De Investigaciones Científicas**
**28006 Madrid (ES)**

(72) Inventors:
• **GARCES, Rafael**
**E-41907 Valencia de la Concepción (ES)**
• **MARTINEZ-FORCE, Enrique**
**E-41012 Sevilla (ES)**

(74) Representative: **Van Someren, Petronella F. H. M.**
**Arnold & Siedsma**
**Sweelinckplein 1**
**2517 GK Den Haag (NL)**

(56) References cited:
**WO-A-00/74470    WO-A-02/065829**

• **FERNANDEZ-MOYA V ET AL: "Identification of triacylglycerol species from high-saturated sunflower (Helianthus annuus) mutants" JOURNAL OF AGRICULTURAL AND FOOD CHEMISTRY, AMERICAN CHEMICAL SOCIETY. WASHINGTON, US, vol. 48, no. 3, March 2000 (2000-03), pages 764-769, XP002233189 ISSN: 0021-8561**
• **OSORIO J ET AL: "MUTANT SUNFLOWERS WITH HIGH CONCENTRATION OF SATURATED FATTY ACIDS IN THE OIL" CROP SCIENCE, CROP SCIENCE SOCIETY OF AMERICA, MADISON, WI, US, vol. 35, 1995, pages 739-742, XP000677842 ISSN: 0011-183X cited in the application**
• **FERNANDEZ-MOYA VALLE ET AL: "Temperature-related non-homogeneous fatty acid desaturation in sunflower (Helianthus annuus L.) seeds." PLANTA (BERLIN), vol. 216, no. 5, March 2003 (2003-03), pages 834-840, XP002291684 ISSN: 0032-0935 cited in the application**
• **MARTINEZ-FORCE ENRIQUE ET AL: "Fatty acid composition in developing high saturated sunflower (Helianthus annuus) seeds: Maturation changes and temperature effect" JOURNAL OF AGRICULTURAL AND FOOD CHEMISTRY, vol. 46, no. 9, September 1998 (1998-09), pages 3577-3582, XP002291887 ISSN: 0021-8561**
• **PEREZ-VICH BEGONA ET AL: "Inheritance of medium stearic acid content in the seed oil of a sunflower mutant CAS-4." CROP SCIENCE, vol. 42, no. 6, 2002, pages 1806-1811, XP002291685 ISSN: 0011-183X**

- **RESKE J ET AL: "TRIACYLGLYCEROL COMPOSITION AND STRUCTURE IN GENETICALLY MODIFIED SUNFLOWER AND SOYBEAN OILS" JOURNAL OF THE AMERICAN OIL CHEMISTS' SOCIETY, AMERICAN OIL CHEMISTS' SOCIETY. CHAMPAIGN, US, vol. 74, no. 8, 1997, pages 989-998, XP008014394 ISSN: 0003-021X cited in the application**
- **DAMIANI P ET AL: "Stereospecific analysis of triacylglycerols from vegetable oils by two procedures: II. Normal and high-oleic sunflower oils" JOURNAL OF THE AMERICAN OIL CHEMISTS' SOCIETY, vol. 74, no. 8, 1997, pages 927-933, XP002076930 ISSN: 0003-021X**
- **LIU QING ET AL: "High-stearic and high-oleic cottonseed oils produced by hairpin RNA-mediated post-transcriptional gene silencing" PLANT PHYSIOLOGY (ROCKVILLE), vol. 129, no. 4, August 2002 (2002-08), pages 1732-1743, XP002291940 ISSN: 0032-0889**
- **CANTISANS S ET AL: "ENZYMATIC STUDIES OF HIGH STEARIC ACID SUNFLOWER SEED MUTANTS" PLANT PHYSIOLOGY AND BIOCHEMISTRY, GAUTHIER-VILLARS, PARIS, FR, vol. 38, no. 5, 28 January 2000 (2000-01-28), pages 377-382, XP000960646 ISSN: 0981-9428**

Remarks:
    The file contains technical information submitted after the application was filed and not included in this specification

**EP 1 689 222 B1**

**Description**

OBJECT OF THE INVENTION

The object of the present invention is a sunflower oil directly obtained from sunflower seeds with 12-40.8% of stearic acid referred to the total fatty acid content and with a modified fatty acid distribution between positions *sn*-1 and *sn*-3 of the triacylglycerol (TAG) molecule as compared to oil obtained from wild type sunflower seeds. The invention also relates to a sunflower plant and seeds containing an endogenous oil with the characteristics mentioned. .

BACKGROUND ART

Oil and fats are made mainly from triglycerides, these are molecules formed by a glycerol backbone and three fatty acids esterified to the three hydroxyls groups of the glycerol (Gunstone et al. 1994). The chemical and physical, and also, the nutritional properties of oils are determined by the fatty acid composition of oils and the distribution of these fatty acids in the different triglyceride species. The three stereochemical positions of the fatty acids are denominated *sn*-1, *sn*-2 and *sn*-3. The fact that an oil is solid at a specific temperature or has a good stability is correlated with a reduced level of double bonds in the fatty acids. The main fatty acids found in oilseeds are the linoleic acid (18:2) with 18 carbon atoms and two double bonds and the oleic acid (18:1) with only one double bond, making these oils liquid at room temperature. Some oils, like the one from soybean and canola, have also linolenic acid (18:3) with 18 carbons and 3 double bonds. Those fatty acids are unsaturated because they have one or more double bonds. The vegetable oils also have minor amounts of saturated fatty acids, without any double bond, like palmitic acid that has 16 carbons (16:0), stearic acid with 18 carbons (18:O) arachidic acid with 20 carbons (20:0) and behenic acid (22:0) with 22 carbons.
The unsaturated fatty acids are beneficial to health and the saturated fatty acids neutral or unhealthy, depending on the fatty acid and the position in the triglyceride molecule. On the other hand some tropical vegetable oils and animal fats have short and medium chain saturated fatty acids like lauric acid with 12 carbons (12:0) and myristic acid, a saturated fatty acid with 14 carbons (14:0), being the last one the worst for health. Palmitic acid and stearic acid are the usual saturated fatty acids found in temperate vegetable oils (Table 1). Palmitic acid is considered a little unhealthy and stearic acid is considered neutral.
However, it is very important to consider a second property that depends on the position of the fatty acids in the triglyceride molecule. A saturated long chain fatty acid is less harmful if it is not bound to the middle position (sn-2) of the glycerol. During fat digestion the pancreatic lipase hydrolyzes the fatty acids found in the *sn*-1 and *sn*-3 positions of the glycerol. While the fatty acid in the middle position is kept bound to the glycerol forming a monoglyceride that has detergent properties and is assimilated perfectly, the fatty acids liberated from the positions *sn*-1 and *sn*-3 react with calcium or magnesium producing insoluble salt with these metals, making the intestinal adsorption very difficult. As a result thereof they are excreted. As shown in table 1, all saturated fatty acids of vegetable oils, with the exception of palm oil, are not located in position *sn*-2, for this reason they do not negatively affect the cholesterol level, although they have a high palmitic content like cocoa butter or medium palmitic like olive oil.

**Table 1**

| Fatty acid composition of edible fat and oils (Álvarez-Ortega et al. 1997; Chow 1992; Gunstone et al. 1994). | | | | | | | | |
|---|---|---|---|---|---|---|---|---|
| Fat or oil | Fatty acid composition (%) | | | | | | | |
| | ≤12:0 | 14:0 | 16:0 | 16:1 | 18:0 | 18:1 | 18:2 | *Trans* | Saturated in *sn*-2 |
| Lard | | 2 | 25 | 3 | 12 | 45 | 10 | 1 | 79 |
| Butter | 12 | 10 | 26 | 2 | 12 | 28 | 3 | 3 | 84 |
| Palm | | 1 | 45 | | 5 | 39 | 9 | | 18 |
| Olive | | | 14 | 1 | 3 | 71 | 10 | | 2 |
| Cocoa | | | 26 | | 35 | 35 | 3 | | 4 |
| Sunflower | | | 7 | | 5 | 30 | 57 | | 1 |
| Sunflower High Oleic (HO) | | | 5 | | 4 | 88 | 2 | | 1 |

For many food preparations the food industry needs plastic or solid fats (such as animal fats) with good stability. Bakery, pastry and, of course, margarine and spreads, require solid fat, while deep frying industry wants liquid oil resistant to thermo-oxidation. In the eighties, the food industry following nutrition expert recommendations and consumer demands switched from animal fats to vegetable oils. These oils do not have the appropriate properties to be used in these food preparations; they must be chemically modified through partial hydrogenation and/or trans-esterification. Hydrogenation reduces the doubles bounds of the unsaturated fatty acids with hydrogen and a heavy metal as a catalyst. During this process the saturated fatty acids increase, but at the same time the number of artificial fatty acid isomers *cis* and trans increases. The *trans* isomers, although they are unsaturated fatty acids, have physical properties similar to saturated fatty acids. The main problem with these *trans* fatty acids is that they are even worse than the animal saturated fatty acids with respect to cholesterol levels, and they are involved in some essential fatty acid deficiencies or in certain cancers, like women's breast cancer.

Chemical trans-esterification leads to a redistribution of all fatty acids within the triglyceride molecules; later by fractionation a portion enriched in saturated triglycerides can be obtained. Through this process a healthy vegetable oil is converted into an unhealthy fat, like lard, with a lot of saturated fatty acids in position *sn*-2. This oil will increase the low density (bad) cholesterol. In conclusion, the processes used to chemically modify the vegetable oils are not particularly healthy, changing the properties of these oils in such a way that the new ones are less healthy. Taking into account the technological and nutritional data, the best oil should be a natural vegetable oil with an increased content of stearic acid as saturated fatty acid, preferably bound to the glycerol backbone through the *sn*-1 and *sn*-3 positions, and oleic or linoleic acids as unsaturated fatty acids which are bound to the three *sn* positions.

Several fatty acid sunflower mutant lines were selected and fixed after a mutagenesis program (Osorio et al. 1995). Some of these mutants have a high content of saturated fatty acids in the seed oil: CAS-3 with a least 26% of stearic acid; CAS-4 and CAS-8 with at least medium levels of stearic acid (12-16%). This material and other like CAS-29, 30 and 31, selected after biochemical studies and further recombination, make a wide germplasm collection **(Table 2).**

**Table 2**

| Fatty acid composition of selected sunflower material from the sunflower collection of Instituto de la Grasa, CSIC, Seville, Spain | | | | | | | |
|---|---|---|---|---|---|---|---|
| Oil type | Line | Fatty acid composition (mol %) | | | | | |
| | | 16:0 | 18:0 | 18:1 | 18:2 | 20:0 | 22:0 |
| Medium 18:0 | CAS-4 | 5.9 | 11.9 | 27.8 | 53 | 0.6 | 0.7 |
| High 18:0 | CAS-3 | 5.4 | 26.1 | 14.2 | 51.3 | 1.4 | 1.3 |
| High 18:0 | CAS-14 | 8.4 | 37.3 | 12.4 | 37.9 | 2.2 | 1.8 |
| High 18:0 | CAS-30 | 5.8 | 32.1 | 9.4 | 49.3 | 1.9 | 1.5 |
| High 18:0 | CAS-29 | 6.7 | 31.9 | 21.2 | 36.2 | 1.8 | 2.2 |
| High 18:0 | CAS-31 | 7.2 | 31.7 | 14.7 | 43.3 | 1.4 | 1.7 |
| High 18:0 | CAS-15 | 5.2 | 26.3 | 57.8 | 7.2 | 1.2 | 1.9 |
| High 18:0 | CAS-26 | 10.1 | 23.3 | 59.1 | 4.5 | 1.2 | 1.8 |
| High 18:0 | CAS-24 | 7.2 | 24.5 | 60.6 | 4.4 | 1.4 | 1.9 |

The genetic characterization of the mutants has shown that the inheritance of the altered fatty acid levels is gametophytic and controlled by alleles at a reduced number of loci, making feasible their transference to target inbred lines in few backcross cycles. The study of the temporal and spatial expression of these mutant characters showed that the mutant characters are expressed only during seed formation, are little influenced by growth temperature and are not expressed in the vegetative tissues. These sunflower mutant lines do not have any collateral negative effect like the ones found in arabidopsis and canola high stearic mutants.

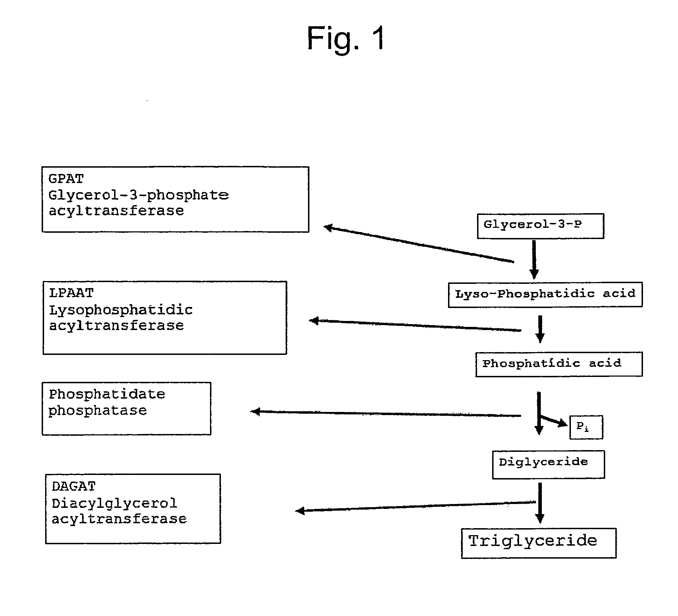

Plant triglycerides are produced by the glycerol-3-P pathway (Kennedy pathway). Initially **(Figure 1),** two acylations of glycerol 3-phosphate in *sn*-1 and *sn*-2 positions with acyl-CoA esters occurs, producing phosphatidate by the enzymes, glycerol 3-phosphate acyltransferase (GPAT) and lysophosphatidate acyltransferase (LPAAT), respectively. The phosphatidate is then hydrolysed to diglyceride by the phosphatidate phosphohydrolase and, subsequently, the diglyceride can be further acylated by acyl-CoA to yield triglyceride (a reaction catalysed by the diglyceride acyltransferase, DAGAT). The last enzyme is exclusive to the triglyceride biosynthesis. Those acyltransferases regulate fatty acid stereochemical distribution.

During the analysis of the sunflower mutant triglyceride composition, 38 different molecular species were found (Fernández-Moya et al. 2000). But unexpectedly, the triglycerides synthesized by the high stearic lines do not have a random distribution in the positions sn-1 and *sn*-3 as expected by the Vander Wal (1960) theory that supposedly was settled for all oils. The enzymes responsible for this unusual distribution are the acyltransferases that synthesized the triglycerides from the acyl-CoA pool and glycerol-3-P. Taking into account that in vegetable systems the triglyceride synthesis implies that no saturated fatty acid will be bound to the *sn*-2 position of the glycerol (Álvarez-Ortega et al;. 1997), the specific enzymes responsible for this effect should be the glycerol-3P acyltransferase and/or the diglyceride acyltransferase.

In the research that led to the invention a mathematical coefficient, alpha S ($\alpha$S), has been developed which calculates the relative distribution of saturated fatty acid in TAG *sn*-1 and *sn*-3 positions. The value of $\alpha$S goes from 0, meaning one of the positions without any saturated fatty acid, to 0.5, both positions having the same saturated fatty acid content. If a triglyceride distribution is made according to the Vander Wal theory, then $\alpha$ = 0.5 and the different fatty acids are distributed evenly in the triglyceride. This is very important in the case of the saturated fatty acids distribution between positions *sn*-1 and *sn*-3, because if there is more saturated fatty acids in one of these positions $\alpha$ is smaller than 0.5 and the amount of disaturated triglycerides is smaller than theoretically expected. That is exactly what the inventors found in sunflower oils, mainly in the ones with stearic acid higher than 12%. The maximum amount of disaturated triglycerides, which are advantageous for making plastic fats for spreads, margarines, shortening, bakery, pastry, etc., is obtained when $\alpha$ = 0.5, the smaller this value of $\alpha$ is in the saturated fatty acid distribution in positions *sn*-1 and sn-3 of the triglyceride molecule, the worst this oil is for this specific food purposes. Thus, for particular uses mutant sunflowers that have an advantageous relative distribution of saturated fatty acid in TAG *sn*-1 and *sn*-3 positions can be selected based on calculation of the value $\alpha$.

This coefficient was calculated knowing the total saturated fatty acid composition of the triglyceride (S), the saturated fatty acid composition in *sn*-2 ($S_2$)' which two can be calculated according to Álvarez-Ortega et al. (1997), and the triglyceride molecular species composition, which can be calculated according to Fernández-Moya et al. (2000).

**Table 3** Saturated and unsaturated fatty acid percentage in each TAG position ($S_1$, $S_2$ and $S_3$) in function of S, $S_2$ and $\alpha$S: Total saturated fatty acid content; $S_2$: *sn*-2 saturated fatty acid content; $\alpha$: distribution coefficient of saturated fatty acid between *sn*-1 and *sn*-3 positions.

| | TAG positions | | |
| --- | --- | --- | --- |
| | 1 | 2 | 3 |
| Saturated(S) | $(3S-S_2)\,\alpha$ <br> $(S_1)$ | $S_2$ | $(3S-S_2)\,(1-\alpha)$ <br> $(S_3)$ |
| Unsaturated (U) | $100-[(3S-S_2)\alpha]$ <br> $(U_1)$ | $100-S_2$ <br> $(U_2)$ | $100-[(3S-S_2)\,(1-\alpha)]$ <br> $(U_3)$ |

The percentages of the different TAG subclasses (Trisaturated, SSS; Disaturated, SUS; Monosaturated , SUU; and Triunsaturated, UUU) are usually calculated using the following formulas:

$$\text{SSS (\%)} = S_1 S_2 S_3 / 10000 \quad \text{(i)}$$

$$\text{SUS (\%)} = (U_1 S_2 S_3 + S_1 U_2 S_3 + S_1 S_2 U_3) / 10000 \quad \text{(ii)}$$

$$\text{SUU (\%)} = (S_1 U_2 U_3 + U_1 S_2 U_3 + U_1 U_2 S_3) / 1000 \quad \text{(iii)}$$

$$\text{UUU (\%)} = U_1 U_2 U_3 / 10000 \quad \text{(iv)}$$

Using the values given for $S_1$, $S_2$, $S_3$, $U_1$, $U_2$ and $U_3$ in Table 3 we can calculate the value for distribution coefficient $\alpha$ by the following reasoning in the different TAG:

a) From trisaturated TAG species (SSS):

$$\text{SSS } (\%) = S_1 S_2 S_3 / 10000 \quad \text{(i)}$$

Substituting the values for $S_1$, $S_2$ and $S_3$ from **Table 3,** we obtain:

$$S_1 S_2 S_3 = [(2S - S_2)\alpha] \times S_2 \times [(3S - S_2)(1 - \alpha] =$$

$$(3S\alpha - S_2\alpha) \times S_2 \times (3S - 3S\alpha - S_2 - S_2\alpha) =$$

$$3SS_2\alpha - S_2{}^2\alpha) \times (3S - 3S\alpha - S_2 - S_2\alpha) =$$

$$9S^2S_2\alpha - 9S^2S_2\alpha^2 - 3SS_2{}^2\alpha + 3SS_2{}^2\alpha^2 - 3SS_2{}^2\alpha + 3SS_2{}^2\alpha^2 + S_2{}^3\alpha - S_2{}^3\alpha^2 =$$

$$(-9S^2S_2 + 6SS_2{}^2 - S_2{}^3)\alpha^2 + (9S^2S_2 - 6SS_2{}^2 + S_2{}^3)\alpha \quad \text{(v)}$$

Rearranging in (i),

$$S_1 S_2 S_3 - 10000SSS(\%) = 0$$

Substituting for $S_1 S_2 S_3$ value from equation (v),

$$(-9S^2S_2 + 6SS_2{}^2 - S_2{}^3)\alpha^2 + (9S^2S_2 - 6SS_2{}^2 + S_2{}^3)\alpha - 10000SSS (\%) = 0$$

$\alpha$ value is calculated as a

$$\alpha = \frac{-b \pm \sqrt{b^2 - 4ac}}{2a} = \frac{-b}{2a} \pm \frac{\sqrt{b^2 - 4ac}}{2a} = 0.5 \pm \frac{\sqrt{a^2 - 4ac}}{2a}$$

quadratic equation
($ax^2 + bx + c = 0$) that can be simplified because $a = -b$
where $a = -9S^2S_2 + 6SS_2{}^2 - S_2{}^3$, $b = 9S^2S_2 - 6SS_2{}^2 + S_2{}^3$ and $c = 10000SSS$ (%), SSS(%) being the total amount of trisaturated TAGs in the seed/oil.

b) From disaturated TAG species (SUS):

$$\text{SUS } (\%) = U_1 S_2 S_3 + S_1 U_2 S_3 + S_1 S_2 U_3) / 10000 \quad \text{(ii)}$$

Subsituting the values for $S_1$, $S_2$, $S_3$, $U_1$, $U_2$ and $U_3$ from Table 3, we obtain:

$$U_1 S_2 S_3 = \{100 - [3S - S_2)\alpha]\} \times S_2 \times [(3S - S_2)(1 - \alpha)] =$$

$$300SS_2 - 300SS_2\alpha - 100S_2{}^2 + 100S_2{}^2\alpha - 9S^2S_2\alpha + 9S^2S_2\alpha^2 + 3SS_2{}^2\alpha -$$

$$3SS_2{}^2\alpha^2 + 3SS_2{}^2\alpha - 3SS_2{}^2\alpha^2 - S_2{}^3\alpha + S_2{}^3\alpha^2 \quad \text{(vi)}$$

$$S_1U_2S_3 = (3S - S_2)\alpha \times (100 - S_2) \times [(3S - S_2)(1 - \alpha)] =$$
$$900S^2\alpha - 300SS_2\alpha - 9S^2S_2\alpha + 3SS_2^2\alpha - 900S^2\alpha^2 + 300SS_2\alpha^2 + 9S^2S_2\alpha^2 -$$
$$3SS_2^2\alpha^2 - 300SS_2\alpha + 100S_2^2\alpha + 3SS_2^2\alpha - S_2^3\alpha + 300SS_2\alpha^2 - 100S_2^2\alpha^2 -$$
$$3SS_2^2\alpha^2 + S_2^3\alpha^2 \quad (vii)$$

$$S_1S_2U_3 = [(3S - S_2)\alpha] \times S_2 \times \{100 - [(3S - S_2)(1 - \alpha)]\} =$$
$$300SS_2\alpha - 9S^2S_2\alpha + 9S^2S_2\alpha^2 + 3SS_2^2\alpha - 3SS_2^2\alpha^2 - 100S_2^2\alpha +$$
$$3SS_2^2\alpha - 3SS_2^2\alpha^2 - S_2^3\alpha + S_2^3\alpha^2 \quad (viii)$$

Rearranging in (ii),

$$(U_1S_2S_3 + S_1U_2S_3 + S_1S_2U_3) - 10000SUS\,(\%) = 0$$

Substituting for $U_1S_2S_3$, $S_1U_2S_3$ and $S_1S_2U_3$ values from equations (vi), (vii) and (viii) respectively, and grouping in function of $\alpha$:

$$(600SS_2 - 18SS_2^2 + 27S^2S_2 - 900S^2 - 100S_2^2 + 3S_2^3)\alpha^2 + (-600SS_2 +$$
$$18SS_2^2 - 27S^2S_2 + 900S^2 + 100S_2^2 - 3S_2^3)\alpha + 300SS_2 - 100S_2^2 -$$
$$10000SUS\,(\%) = 0$$

$\alpha$ value is calculated as a quadratic equation that can be simplified because a = -b

$$\alpha = \frac{-b \pm \sqrt{b^2 - 4ac}}{2a} = \frac{-b}{2a} \pm \frac{\sqrt{b^2 - 4ac}}{2a} = 0.5 \pm \frac{\sqrt{a^2 - 4ac}}{2a}$$

where a = $600SS_2 - 18SS_2^2 + 27S^2S_2 - 900S^2 - 100S_2^2 + 3S_2^3$ and c = $300SS_2 - 100S_2^2 - 10000SUS(\%)$, SUS(%) being the total amount of disaturated TAGs in the seed/oil.

c) From monounsaturated TAG species (SUU):

$$SUU\,(\%) = (S_1U_2U_3 + U_1S_2U_3 + U_1U_2S_3)/10000 \quad (iii)$$

Substituting the values for $S_1$, $S_2$, $S_3$, $U_1$, $U_2$ and $U_3$ from **Table 3**, we obtain:

$$S_1U_2U_3 = 30000S\alpha - 900S^2\alpha + 900S^2\alpha^2 + 300SS_2\alpha - 300SS_2\alpha^2 -$$
$$10000S_2\alpha + 300SS_2\alpha - 300SS_2\alpha^2 - 100S_2^2\alpha + 100S_2^2\alpha^2 - 300SS_2\alpha +$$
$$9S^2S_2\alpha - 9S^2S_2\alpha^2 - 3SS_2^2\alpha + 3SS_2^2\alpha^2 + 100S_2^2\alpha - 3SS_2^2\alpha + 3SS_2^2\alpha^2 +$$
$$S_2^3\alpha - S_2^3\alpha^2 \quad (ix)$$

$$U_1S_2U_3 = 10000S_2 - 300SS_2 + 300SS_2\alpha + 100S_2^2 - 100S_2^2\alpha -$$
$$300SS_2\alpha + 9S^2S_2\alpha - 9S^2S_2\alpha^2 - 3SS_2^2\alpha + 3SS_2^2\alpha^2 + 100S_2^2\alpha -$$
$$3SS_2^2\alpha + 3SS_2^2\alpha^2 + S_2^3\alpha - S_2^3\alpha^2 \quad (x)$$

$$U_1U_2S_3 = 30000S - 10000S_2 - 30000S\alpha + 10000S_2\alpha - 300SS_2 +$$
$$100S_2^2 + 300SS_2\alpha - 100S_2^2\alpha - 900S^2\alpha + 300SS_2\alpha + 900S^2\alpha^2 -$$
$$300SS_2\alpha^2 + 9S^2S_2\alpha - 3SS_2^2\alpha - 9S^2S_2\alpha^2 + 3SS_2^2\alpha^2 + 300SS_2\alpha -$$

$$100S_2^2\alpha - 300SS_2\alpha^2 + 100S_2^2\alpha^2 - 3SS_2^2\alpha + S_2^3\alpha + 3SS_2^2\alpha^2 - S_2^3\alpha^2$$
$$(xi)$$

Rearranging in (iii):

$$(S_1U_2U_3 + U_1S_2U_3 + U_1U_2S_3) - 10000SUU \ (\%) = 0$$

Substituting for $S_1U_2U_3$, $U_1S_2U_3$ and $U_1U_2S_3$ values from equations (ix), (x) and (xi) respectively, and grouping in function of $\alpha$:

$$(-1200SS_2 + 18SS_2^2 - 27S^2S_2 + 1800S^2 + 200S_2^2 - 3S_2^3)\alpha^2 +$$
$$(1200SS_2 - 18SS_2^2 + 27S^2S_2 - 1800S^2 - 200S_2^2 + 3S_2^3)\alpha -$$
$$600SS_2 + 200S_2^2 + 30000S - 10000SUU(\%) = 0$$

$\alpha$ value is calculated as a quadratic equation that can be simplified because a = -b

$$\alpha = \frac{-b \pm \sqrt{b^2 - 4ac}}{2a} = \frac{-b}{2a} \pm \frac{\sqrt{b^2 - 4ac}}{2a} = 0.5 \pm \frac{\sqrt{a^2 - 4ac}}{2a}$$

where a = **-1200SS$_2$ + 18SS$_2$$^2$ - 27S$^2$S$_2$ + 1800S$^2$ + 200S$_2$$^2$ - 3S$_2$$^3$** and c = **-600SS$_2$ + 200S$_2$$^2$ + 30000S - 10000SUU**

**(%),** SUU(%) being the total amount of monosaturated TAGs in the seed/oil.

d) From triunsaturated TAG species:

$$UUU(\%) = U_1U_2U_3/10000 \quad (iv)$$

Substituting the values for $U_1$, $U_2$ and $U_3$ from Table 3, we obtain:

$$U_1U_2U_3 = (600SS_2 - 6SS_2{}^2 + 9S^2S_2 - 900S^2 - 100S_2{}^2 + S_2{}^3)\alpha^2 +$$
$$(-600SS_2 + 6SS_2{}^2 - 9S^2S_2 + 900S^2 + 100S_2{}^2 - S_2{}^3)\alpha + 300SS_2 -$$
$$100S_2{}^2 - 30000S + 1000000 \quad (xii)$$

Rearranging in (iv):

$$U_1U_2U_3 - 10000UUU(\%) = 0$$

Substituting for $U_1U_2U_3$ value from equation (xii), and grouping in function of $\alpha$:

$$(600SS_2 - 6SS_2{}^2 + 9S^2S_2 - 900S^2 - 100S_2{}^2 + S_2{}^3)\alpha^2 + (-600SS_2 +$$
$$6SS_2{}^2 - 9S^2S_2 + 900S^2 + 100S_2{}^2 - S_2{}^3)\alpha + 300SS_2 - 100S_2{}^2 -$$
$$30000S + 1000000 - 10000UUU(\%) = 0$$

$\alpha$ value is calculated as a quadratic equation that can be simplified because a = -b

$$\alpha = \frac{-b \pm \sqrt{b^2 - 4ac}}{2a} = \frac{-b}{2a} \pm \frac{\sqrt{b^2 - 4ac}}{2a} = 0.5 \pm \frac{\sqrt{a^2 - 4ac}}{2a}$$

where a = **$600SS_2 - 6SS_2{}^2 + 9S^2S_2 - 900S^2 - 100S_2{}^2 + S_2{}^3$**, and c = **$300SS_2 - 100S_2{}^2 - 30000S + 1000000 - 10000UUU(\%)$,** UUU(%) being the total amount of triunsaturated TAGs in the seed/oil.

To avoid deviations by experimental errors in the determination by GLC of TAG species we define $\alpha$s as the weighted average of $\alpha$ values calculated from SSS ($\alpha$SSS), from SUS ($\alpha$SUS), from SUU ($\alpha$SUU) and from UUU ($\alpha$UUU).

$$\alpha S = \frac{(\alpha SSS \times SSS(\%)) + (\alpha SUS \times SUS(\%)) + (\alpha SUU \times SUU(\%)) + (\alpha UUU \times UUU(\%))}{(SSS(\%) + SUS(\%) + SUU(\%) + UUU(\%))}$$

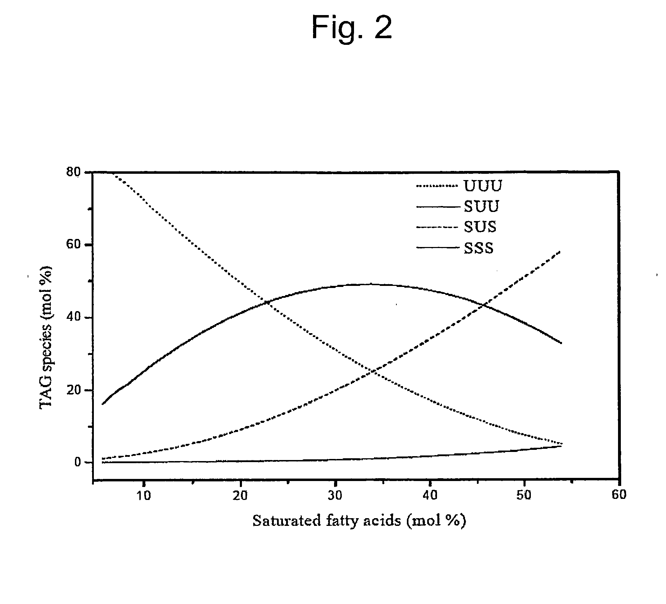

In a random distribution of the saturated fatty acid between positions *sn*-1 and *sn*-3 of the triglyceride molecule 50% of each saturated fatty acid should be in each position, being the optimum to have the maximum SUS triglyceride molecules, S being a saturated and U an unsaturated fatty acid, respectively. **Figure 2** shows the proportion of the different TAG species in sunflower oil with increasing saturated fatty acid content if a random distribution between *sn*-1 and *sn*-3 occurs. Those curves have been generated substituting $\alpha$ for 0.5, using the content of sunflower saturated fatty acids in *sn*-2 position (Álvarez-Ortega et al. 1997) and increasing values for total saturated fatty acid content in the formulas (i), (ii), (iii) and (iv).

The α coefficient of an oil could also be calculated by chemically analyzing the fatty acid composition of the three *sn* positions of the TAG molecule. This analysis could be made following the methods proposed by Laakso and Christie (1990) or Takagi and Ando (1991). These methods make it possible to know the fatty acid content of the three sn positions, but need a large size sample and could not be applied to a small sample, like a half-seed as in our method. In this case the formula is as follows, αs being the smallest of these two values, except when both are 0.5. In this case α = 0.5.

$$\alpha S = Min\left(\frac{S_1}{S_1 + S_3}; \frac{S_3}{S_1 + S_3}\right)$$

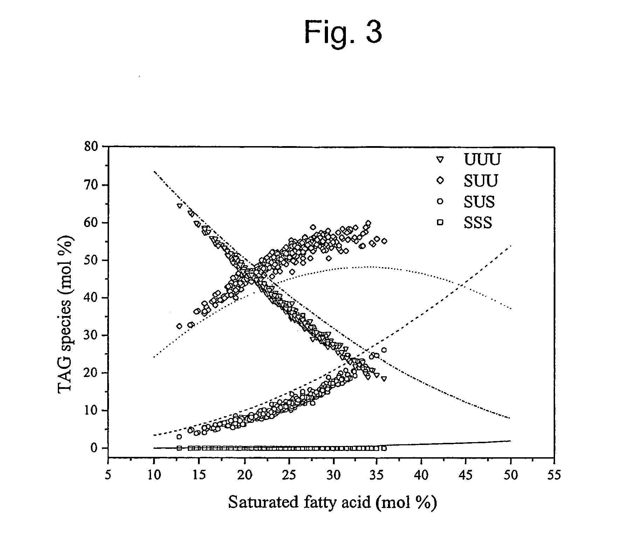

The inventors have studied this distribution in TAG in actual sunflower oils. The data show, as expected, that saturated fatty acids (S) are located mainly in positions *sn*-1 and *sn*-3 of the glycerol molecule in normal and high saturated sunflower oils and in very low amount in *sn*-2. The main fatty acids in this position are oleic and linoleic acids as expected and in accordance with the data of Álvarez-Ortega et al. (1997). However, the acyl groups were not distributed according to the 1,3-random, 2-random theory (Vander Wal, 1960). The saturated fatty acids, palmitic and stearic, were not evenly distributed. These results are in agreement with previous data (Reske et al., 1997) that show a preference for position *sn*-3 over the position *sn*-1 of saturated fatty acids, mainly when stearic content was increased (11%) with respect to commodity sunflower that had 4.8%. The TAG distribution of sunflower oils differing in the stearic acid content and in the oleic/linoleic ratio, from high oleic to high linoleic is shown in **Figure 3**. It was found that the theoretical values of sunflower TAG species groups (SSS, SUS, SUU and UUU) expected for different saturated fatty acid content, based on the observed composition in position *sn*-2 and total fatty acid content applying the 1,3-random 2-random theory, are different from the TAG compositions found in the analyzed seeds. The TAG composition has been determined by GLC and the data of these TAG species grouped by level of unsaturation (Fernández-Moya et al., 2000).
As Figure 3 shows, sunflower saturated fatty acids follow an asymmetric distribution in TAG, the obtained values for SUU always being higher than the expected values and the values for SUS and UUU lower than the anticipated values by a non-specific distribution in positions *sn*-1 and *sn*-3.
These results are also in agreement with previous results with high stearic sunflower mutant TAG species containing two molecules of linoleic acid and one saturated fatty acid that were more abundant than expected by the 1,3-random 2-random theory (Fernández-Moya et al., 2000). The increment of SUU and the reduction of UUU TAG species were directly correlated with the total stearic acid content in the oil.
The distribution coefficient of the saturated fatty acids (α) between positions *sn*-1 and *sn*-3 in control and high stearic mutant lines has been calculated (**Table 4**). This coefficient is always between 0.19 and 0.37 when the linoleic acid content is higher than the oleic acid content and between 0.15 and 0.27 when the oleic acid content is higher than the linoleic acid content.

**Table 4**

Stearic content (18:0), total saturated fatty acid content (S), the different TAG groups (SUS, SUU and UUU), and the value of the distribution coefficient α in several normal and mutant lines of sunflower are shown. RHA-274 is from USDA-ARS, Northerm Crop Science Lab, Fargo, ND. Other lines are from Sunflower Collection of Instituto de la Grasa, CSIC, Seville, Spain. The content of the different fatty acids in the lines are represented as: HS, high stearic; MS, medium stearic; HL, high linoleic and HO, high oleic.

| Line | Type | 18:0 | S | SUS | SUU | UUU | α |
|---|---|---|---|---|---|---|---|
| RHA-274 | Normal | 5 | 11.7 | 2.9 | 29.5 | 67.7 | 0.31 |
| CAS-3 | HSHL | 25.6 | 37 | 28.6 | 48.9 | 21.6 | 0.33 |
| CAS-29 | HSHL | 33.2 | 42 | 28.9 | 55.8 | 14.7 | 0.37 |
| CAS-4 | MSHL | 12.9 | 20.3 | 8.3 | 44.5 | 47.3 | 0.29 |
| G-8 | HO | 4.9 | 9.4 | 1.1 | 22.7 | 76.2 | 0.19 |
| CAS-15 | HSHO | 23.4 | 33.5 | 19.3 | 61.9 | 18.8 | 0.23 |
| DG-9 | MSHO | 19.1 | 29.8 | 16.8 | 55.6 | 27.5 | 0.27 |

Therefore there is still a need for a sunflower oil that has a distribution coefficient above 0.38.

SUMMARY OF THE INVENTION

The object of the present invention is provision of a sunflower oil directly obtained from sunflower seeds with 12-40.8%, preferably at least 20% of stearic acid referred to the total fatty acid content, and in said oil the distribution coefficient of saturated fatty acids α between positions *sn*-1 and *sn*-3 of the TAG molecule is at least 0.28, preferably at least 0.32 and most preferably 0.36, wherein the oleic acid content is higher than the linoleic acid content in-the oil and the stearic acid content is at least 12%, preferably at least 20% referred to the total fatty acid content.

The present invention further relates to sunflower plants that form seeds containing an endogenous oil, obtainable from said sunflower seeds, with the characteristics indicated above and to sunflower seeds produced by said sunflower plant.

It constitutes another object of the present invention to provide hybrid plants and their progeny that have the above saturated fatty acid distribution between positions *sn*-1 and *sn*-3 and other desirable characteristics.

DETAILED DESCRIPTION OF THE INVENTION

The invention thus relates to a sunflower oil directly obtained from sunflower seeds with a stearic acid content of 12-40.8% referred to the total fatty acid content and where the oleic acid content is higher than the linoleic acid content in the oil, characterized in that the distribution coefficient of saturated fatty acids α between positions *sn*-1 and *sn*-3 is at least 0.28, which seeds are obtainable by growing sunflower plants from sunflower seeds of a high oleic high stearic line, growing sunflower plants from the sunflower seeds of CAS-36, having ATCC deposit accession number PTA-5041, crossing the lines to produce F1 seed and harvesting the seeds, producing F2 seeds and selecting seeds with more than 0.28 α value, high stearic and more oleic than linoleic acid,
wherein the α coefficient is calculated using the formula

$$\alpha S = \text{Min}\left( \frac{S_1}{S_1 + S_3} , \frac{S_3}{S_1 + S_3} \right)$$

$S_1$ being the saturated fatty acid content, in the TAG sn-1 position, $S_3$ being the saturated fatty acid content in the TAG *sn*-3 position and $\alpha S$ being the smallest of the two values, except when both are 0.5, then α is 0,5.

Preferably the distribution coefficient of saturated fatty acids α between positions *sn*-1 and *sn*-3 is at least 0.32, more preferably at least 0.36.

The invention also relates to sunflower plants which form seeds containing an endogenous oil as defined above and to the seeds produced by these plants.

Also disclosed herein is a method for producing a plant which form seeds containing an endogenous oil with at least 12% of stearic acid referred to the total fatty acid content, where the oleic acid content is higher than the linoleic acid content and where the distribution coefficient of saturated fatty acids α between positions *sn*-1 and *sn*-3 is at least 0.28, which method comprises:

a) providing seeds which contain an oil having a stearic acid content of at least 12% referred to the total fatty acid content in the oil and where the oleic acid content is higher than the linoleic acid content;
b) providing seeds which contain an oil having a distribution coefficient α higher than 0.28 in the oil;
c) crossing plants from the seeds provided in step a) and b);
d) harvesting the F1 seed progeny;
e) planting the F1 progeny seeds to grow plants;
f) self-pollinating the plants thus grown to produce F2 seed;
g) testing the seeds for the presence of a stearic acid content of at least 12%, a higher oleic acid content than the linoleic acid content and a distribution coefficient α of at least 0.28;
h) planting seeds having the desired levels of stearic, oleic and linoleic acids content and distribution coefficient α to grow plants;
i) self-pollinating the plants thus grown to produce F3 seed; and
j) optionally repeating steps g), h) and i) until the desired levels of stearic, oleic and linoleic acids content and distribution coefficient α are fixed.

The seeds which contain an oil with at least 12% of stearic acid are provided by:

a) treatment of sunflower seeds having a stearic acid content of less than 12% with a mutagenic agent, in particular sodium azide or an alkylating agent, more particularly ethyl methane sulfonate;
b) producing plants therefrom which are pollinated to produce seeds;
c) testing the seeds for the desired stearic acid content;
d) providing seeds which contain an oil where the oleic acid content is higher than the linoleic acid content;
e) crossing plants from the seeds tested in step c) and from the seeds provided in step d);
f) harvesting the F1 seed progeny;
g) self-pollinating the plants thus grown to produce F2 seed;
h) testing the seeds for the presence of a stearic acid content of at least 12% and a higher oleic acid content than the linoleic acid content;
i) planting seeds having the desired levels of stearic, oleic and linoleic acid content;
j) self-pollinating the plants thus grown to produce F3 seed; and
k) optionally repeating steps h), i) and j) until the desired levels of stearic, oleic and linoleic acid content are fixed.

The seeds which contain an oil where the distribution coefficient of saturated fatty acids $\alpha$ between positions sn-1 and sn-3 is at least 0.38 are provided by:

a) treatment of sunflower seeds having a distribution coefficient $\alpha$ value smaller than 0.38 with a mutagenic agent, inparticular sodium azide or an alkylating agent, more particularly ethyl methane sulfonate;
b) producing plants therefrom which are pollinated to produce seeds;
c) testing the seeds for the desired distribution coefficient $\alpha$ value;
d) optionally repeating steps b) and c).

The invention further relates to hybrid plants obtainable by crossing a first parent plant resulting from the methods above and a second parent plant having desirable characteristics, and to progeny of the hybrid plant. The second parent plant may also be a plant resulting from the method above.

The invention also relates to the use of the oil in the production of food products and to food products prepared with or containing this oil.

Also disclosed is a new type of sunflower mutant, that has a better $\alpha$ distribution than previous sunflower lines. Thus, this mutant has better properties for the production of margarine, spreads, etc. than the presently available lines (**Figure 3**). The best triglycerides for margarine are the saturated-unsaturated-saturated types (SUS), preferably a triglyceride of the type saturated-oleic-saturated (SOS).

This new mutant line, called CAS-36, obtained by technological methods has been deposited in the ATCC and has been given the accession number PTA-5041. This mutant has the best TAG distribution according to the random theory. Data from oil from a sample of seeds of some CAS-36 plants are shown in **Table 5.**

**Table 5**

| Plant | 18:0 | S | O/L | TAG groups | | | $\alpha$ |
| | | | | SUS | SUU | UUU | |
|---|---|---|---|---|---|---|---|
| Stearic, total saturated fatty acid content (S), oleic/linolenic acid ratio (O/L), the different TAG groups, and the value of the distribution coefficient $\alpha$ in some CAS-36 mutant plants are shown. | | | | | | | |
| CAS-36-A | 28.5 | 37.7 | 0.95 | 32.4 | 41.8 | 23.7 | 0.5 |
| CAS-36-B | 28.4 | 38.8 | 0.15 | 35.1 | 46.2 | 18.7 | 0.5 |
| CAS-36-D | 31.2 | 39.7 | 0.93 | 35.8 | 44.8 | 18.4 | 0.5 |
| CAS-36-E | 38.2 | 47.7 | 0.06 | 49.3 | 44.7 | 6.1 | 0.45 |

In U.S. patent 6,475,548 there is a comparison to a reference spread product made with the untreated oil of WO 95/20313, and a margarine made with the stearin fraction of the oil of WO 95/20313. The spread made with the stearin fraction apparently gave good spreadability at temperatures close to refrigerator temperature, appropriate melting in the mouth and stability. The performance of such a fat blend apparently gave a similar performance of known high quality fast compositions, without non-natural components like hydrogenated fat.

It is well known that stearin fractions of fats can be used in a fat phase of spreads to resolve problems in making spreads. For example in U.S. Pat. No. 4,438,149 spreads were prepared with a fat phase containing less than 70% butter fat. This product was too soft in consistency. But when the stearin fraction of the fat was used, a less expensive and more spreadable product was made. U.S. Patent No. 6,475,548 teaches a method of the preparation of a triglyceride fat suited for structuring a liquid vegetable oil or spreads. The method of the preparation of a triglyceride fat was with a high stearic high oleic sunflower oil (HSHOSF), with at least 12 wt. % of stearic acid residues and at least 40 wt. % of oleic acid residues that is subjected to wet fractionation or a dry fractionation and a stearin fraction was collected. Further the above patent teaches that the stearin fraction of the fat blend was gotten by exposing the starting HSHOSF oil to standard conditions for fractionation, either wet or dry fractionation. The fraction containing >30 wt.% of SUS and <40 wt. % of SUU triglycerides should be collected and fractionation would be stopped when the first 25 wt. % of solid fat has crystallised.

The present invention could likewise be employed in a process of the preparation of a triglyceride fat with a high stearic high oleic sunflower oil (HSHOSF) by a wet fractionation or a dry fractionation and then collecting a stearin fraction. More particularly, preparation of a triglyceride fat with a high stearic high oleic sunflower oil (HSHOSF)with at least 12% of stearic acid referred to the total fatty acid content, characterized in that the distribution coefficient of saturated fatty acids $\alpha$ between positions sn-1 and sn-3 is at least 0.28, is subjected to wet fractionation or a dry fractionation and a stearin fraction is collected.

BRIEF DESCRIPTION OF THE FIGURES

**Figure 1** shows the triglyceride biosynthetic pathway.
**Figure 2** is the theoretical distribution of sunflower TAG species distribution with respect to increasing saturated fatty acid content when the $\alpha$ coefficient value is 0.5. See figure legend for TAG nomenclature.
**Figure 3** shows the triglycerides (TAG) distribution in seeds segregating for high oleic and high stearic characters versus the saturated fatty acid content. Theoretical distribution, as control, is represented as lines and the different oil samples distribution are represented as symbols, see figure legend for TAG nomenclature.

The invention will be further illustrated in the Examples that follow and that are not intended to limit the invention.

## EXAMPLES

**Introduction**

The invention relates to a method for preparing sunflower seeds having an better distribution of saturated fatty acids in the different triglycerides molecular species as compared to wild type seeds. This method includes the step of treating parent seeds with a mutagenic agent during a period of time and in a concentration sufficient to induce one or more mutations in the gene trait involved in triglycerides biosynthesis. This results in an increased production of triglycerides species of the type SUS and lower content of SUU. These mutagenic agents include agents such as sodium azide or an alkylating agent, like ethyl methane sulfonate, but any other mutagenic agent having the same or similar effects may also be used. The treated seeds will contain inheritable genetic changes.

These mutated seeds are then germinated and progeny plants are developed therefrom. To increase the traits in the lines the progeny can be crossed or selfed. The progeny seeds are collected and analyzed. Seeds having the near random or random triglyceride trait can then be crossed to any other line and the trait transferred. Optionally, there can be additional cycles of germination, culturing, and selfing to fix the homozygosity of the traits in the lines and crossing and collection of seeds.

Ethyl methane sulfonate was used as mutagenic agent in Example 1. A sunflower line with a $\alpha$ value higher than 0.4 has been obtained. The original sunflower parent line mutagenised was CAS-10 (Sunflower Collection of Instituto de la Grasa, CSIC, Seville, Spain). The oil of that line has an $\alpha$ value smaller than 0.38. The high oleic material used herein is derived from the Russian researched oleic lines (Soldatov, 1976) having an $\alpha$ value between 0.15 and 0.27.

The high oleic high stearic material used is derived from crosses of the high oleic line with mutant CAS-3 deposited under ATCC accession number 75968, and selecting for high oleic high stearic seeds as described in WO-0074470 "High oleic high stearic plants, seeds and oils". A method for the preparations of the high $\alpha$ value plant with more linoleic than oleic acid and vice versa have been described in the following examples.

## EXAMPLE 1

Seeds were mutagenized with a solution of 70 mM of ethyl methane sulfonate (EMS) in water. The treatment was performed at room temperature during 2 hours while shaking (60 rpm). After mutagenesis the EMS solution was discarded and seeds were washed during 16 hours under tap water.

Treated seeds were germinated in the field and plants were self-pollinated. The seeds collected from these plants were used to select new sunflower lines with modifications in the triglyceride distribution. By using the method of Garcés, R. and Mancha, M. (1993) the seed fatty acid composition and by using the method of Fernández-Moya et al. (2000) the triglyceride composition were determined by gas liquid chromatography.

A first plant with a α value of 0.42 was selected. The progeny was cultivated for five generations wherein the α value increased and the new genetic trait became stably fixed in the genetic material of the seed. This line is called CAS-36 and has a linoleic content that is higher than the oleic acid content. The minimum and the maximum α value of the line were 0.38 and 0.5 respectively.

**Table 6** shows some data of the analysis of seeds from several CAS-36 plants and the necessary data to calculate the α values according with the proposed formula.

**Table 6**

Fatty acid, total saturated (S) and saturated in *sn*-2 position (S2) composition of TAG and TAG composition and α Sat calculated following the formula for some CAS-36 oils.

| CAS-36 plant lines | Fatty acid (mol %) | | | | | | | | TAG (mol %) | | | | |
|---|---|---|---|---|---|---|---|---|---|---|---|---|---|
| | 16:0 | 18:0 | 18:1 | 18:2 | 20:0 | 22:0 | S | $S_2$ | SSS | SUS | SUU | UUU | αSat |
| BU-59 | 8.32 | 28.49 | 30.42 | 31.9 | 0.88 | 0 | 37.69 | 4.16 | 2.17 | 32.38 | 41.79 | 23.66 | 0.5 |
| CB-71 | 6.09 | 28.94 | 28.14 | 34.57 | 1.3 | 0.96 | 37.29 | 4.12 | 0.85 | 32.61 | 44.1 | 22.44 | 0.5 |
| CB-88 | 6.3 | 25.03 | 41.5 | 25.73 | 0.9 | 0.54 | 32.77 | 3.65 | 0 | 24.41 | 49.47 | 26.12 | 0.45 |
| CB-79 | 7.63 | 23.88 | 32.15 | 34.02 | 1.3 | 1.01 | 33.83 | 3.76 | 0 | 26.1 | 49.28 | 24.62 | 0.46 |

### EXAMPLE 2

Sunflower plants were grown from the sunflower seeds of the high oleic high stearic line like the one shown in **Table 2.** Sunflower plants were also grown from the sunflower seeds of CAS-36. The lines were crossed. The plants were assisted by artificial pollination in order to ensure adequate seed production occurred. The F1 seed was produced on the high oleic high stearic line, or vice versa, and harvested. The F2 seeds with more than 0.28 α value, high stearic and more oleic than linoleic acids were selected. Although this produces the oil of the present invention the level of production is limited. Therefore fixed inbred lines evidencing seeds with these α values are desirable.

These homozygous fixed inbred high oleic high stearic lines can then be crossed to form hybrid seed, which will produce F2 seed evidencing the desired oil traits of the present invention. Toward this end the F1 seeds were planted and produced plants were selfed in isolated conditions and F2 seed was produced. The F2 seed was tested for the α value. The remaining portion of the seeds evidencing the trait was employed to grow plants to form F3 seed. The selfing and screening and selection process is repeated to develop the fixed homozygous line with an α value higher than 0.28, having all of them an α value of 0.5 or near 0.5.

Once the trait is fixed similar high oleic lines can be crossed to form hybrid seed having the trait as shown in **Table 7.** According to the invention sunflower plants and seeds from which said oil can be extracted have been obtained by means of a biotechnological process. The α value content is an inheritable trait and is fairly independent from the growing conditions.

**Table 7**

Stearic (18:0), total saturated fatty acid content (S), oleic/linolenic acid ratio (O/L), the different TAG groups, and the value of the distribution coefficient α in lines derived from crosses of CAS-36 mutant line and CAS-15 or CAS-**24** (**Table 2**) high stearic lines are shown. The Oleic versus linoleic content ratios are represented as O/L, being >1 because the oleic content is higher than the linoleic content in the selected lines.

| Sunflower lines | 18:0 | S | O/L | SUS | SUU | UUU | α Sat |
|---|---|---|---|---|---|---|---|
| HO1 | 33.5 | 45.8 | 2.48 | 47.5 | 42.3 | 10.2 | 0.49 |
| HO2 | 25.4 | 35 | 1.45 | 28.5 | 47.8 | 23.6 | 0.5 |
| HO3 | 22.8 | 33.8 | 1.05 | 26.7 | 47.8 | 25.5 | 0.5 |
| HO4 | 20.2 | 28.2 | 10.91 | 18.8 | 47 | 34.2 | 0.5 |
| HO5 | 29.8 | 38.9 | 1.48 | 36.4 | 44 | 19.6 | 0.5 |
| HO6 | 40.8. | 51 | 1.7 | 54.3 | 38.6 | 5.1 | 0.47 |
| HO7 | 31 | 42.3 | 6.19 | 37.8 | 51.3 | 10.9 | 0.41 |
| HO8 | 28.6 | 36.6 | 1.8 | 27.8 | 54.2 | 18 | 0.35 |

## REFERENCES

1. Álvarez-Ortega. R., Cantisán, S., Martínez-Force, E. and Garcés, R. (1997) Characterization of polar and non-polar seed lipid classes from highly saturated fatty acid sunflower mutants. Lipids 32, 833-837.
2. Chow, C. K. (1992) Fatty acids in foods and their health implications. Marcel Dekker, New. York.
3. Fernández-Moya, V., Martínez-Force, E. and Garcés, R. (2000) Identification of triacylglycerol species from high-saturated sunflower (Helianthus annuus) mutants. Agric. Food Chem. 48, 764-769.
4. Garcés, R. and Mancha, M. (1993) One-step lipid extraction and fatty acid methyl esters preparation from fresh plant tissues. Anal. Biochem. 211, 139-143.
5. Gunstone, F.D. Harwood, J.L. and Padley, F.B. (1994) The Lipid Handbook. Chapman and Hall. London.
6. Laakso, P. and Christie, W.W. (1990) Chromatographic resolution of chiral diacylglycerol derivatives: potential in the stereo-specific analysis of triacyl-sn-glycerols. Lipids 25, 349-353.
7. Osorio, J., Fernández-Martínez, J., Mancha, M. and Garcés, R. (1995) Mutant sunflowers with high concentration of saturated fatty acids in the oil. Crop Sci. 35, 739-742.
8. Reske, J., Siebrecht, J. and Hazebroed, J. (1997) Triacylglycerol composition and structure in genetically modified sunflower and soybean oils. J. Am. Oil Chem. Soc. 74, 989-998.
9. Takagi, T. and Ando, Y. (1991) Stereospecific analysis of triacyl-sn-glycerols by chiral HPLC. Lipids 26, 542-547.
10. Vander Wal, R.J. (1960) Calculation of the distribution of the saturated and unsaturated acyl groups in fats, from pancreatic lipase hydrolysis data. J. Am. Oil Chem. Soc. 37, 18-20.
11. Soldatov, K.I. (1976) Chemical mutagenesis in sunflower breeding. pp. 352-357. In: Proc. 7th Int. Sunflower Association. Vlaardingen, The Netherlands

## Claims

1. Sunflower oil directly obtained from sunflower seeds with a stearic acid content of 12-40.8% referred to the total fatty acid content and where the oleic acid content is higher than the linoleic acid content in the oil, **characterized in that** the distribution coefficient of saturated fatty acids α between positions *sn*-1 and *sn*-3 is at least 0.28, which seeds are obtainable by growing sunflower plants from sunflower seeds of a high oleic high stearic line, growing sunflower plants from the sunflower seeds of CAS-36, having ATCC deposit accession number PTA-5041, crossing the lines to produce F1 seed and harvesting the seeds, producing F2 seeds and selecting seeds with more than 0.28 α value, high stearic and more oleic than linoleic acid,
wherein the α coefficient is calculated using the formula

$$\alpha S = Min\left(\frac{S_1}{S_1+S_3} , \frac{S_3}{S_1+S_3}\right)$$

, $S_1$ being the saturated fatty acid content, in the TAG sn-1 position, $S_3$ being the saturated fatty acid content in the TAG *sn*-3 position and $\alpha$S being the smallest of the values, except when both are 0.5, then $\alpha$ is 0.5.

2. Sunflower oil according to claim 1, **characterized in that** the distribution coefficient of saturated fatty acids $\alpha$ between positions *sn*-1 and *sn*-3 is at least 0.32.

3. Sunflower oil according to claim 1 or 2, **characterized in that** the distribution coefficient of saturated fatty acids $\alpha$ between positions *sn*-1 and *sn*-3 is at least 0.36.

4. Sunflower oil according to any one of the claims 1-3, **characterized in that** the stearic acid content referred to the total fatty acid content is at least 20%.

5. Sunflower plant which forms seeds containing an endogenous oil according to claims 1-4.

6. Sunflower seeds produced by sunflower plants according to claim 5.

7. A hybrid sunflower plant the seeds of which contain an endogenous oil with a stearic acid content of 12-40.8% referred to the total fatty acid content and in which oil the oleic acid content is higher than the linoleic acid content and a distribution coefficient of saturated fatty acids $\alpha$ between positions *sn*-1 and *sn*-3 of at least 0.28, which plant is obtainable by crossing a first parent plant as claimed in claim 5 and a second parent plant having desirable characteristics.

8. The hybrid sunflower plant as claimed in claim 7, obtainable by crossing a first parent plant which is a plant as claimed in claim 5 and a second parent plant which is a plant as claimed in claim 5 with other desirable characteristics.

9. Progeny of the sunflower plant claimed in any one of the claims 5 and 7-8 the seeds of which contain an endogenous oil with a stearic acid content of 12-40.8% referred to the total fatty acid content and in which oil the oleic acid content is higher than the linoleic acid content and a distribution coefficient of saturated fatty acids $\alpha$ between positions *sn*-1 and *sn*-3 of at least 0.28.

10. Sunflower oil as claimed in any one of the claims 1-4 for the production of a food product.

11. Food product comprising a sunflower oil as claimed in any one of the claims 1-4.

**Patentansprüche**

1. Unmittelbar aus Sonnenblumensamen erhaltenes Sonnenblumenöl mit einem Stearinsäuregehalt von 12 bis 40,8 %, bezogen auf den gesamten Fettsäuregehalt, und wobei in dem Öl der Ölsäuregehalt größer ist als der Linolsäuregehalt, **dadurch gekennzeichnet, dass** der Verteilungskoeffizient $\alpha$ der gesättigten Fettsäuren zwischen den Positionen sn-1 und sn-3 wenigstens 0,28 beträgt, wobei die Samen erhältlich sind durch Anbau von Sonnenblumenpflanzen aus Sonnenblumensamen einer hoch-Öl-hoch-Stearin-Linie, Anbau von Sonnenblumenpflanzen aus Sonnenblumensamen CAS-36 mit der ATCC Hinterlegungszugangsnummer PTA-5041, Kreuzung der Linien zur Erzeugung von F1 Samen und Ernten der Samen, Herstellung von F2 Samen und Auswahl der Samen mit einem $\alpha$-Wert von größer als 0,28, viel Stearinsäure und mehr Öl- als Linolsäure, wobei der $\alpha$-Koeffizient berechnet wird unter Anwendung der Formel

$$\alpha S = Min\left[\frac{S_1}{S_1 + S_{3'}} \cdot \frac{S_3}{S_1 + S_3}\right],$$

wobei $S_1$ der Gehalt an gesättigten Fettsäuren in der TAG sn-3 Position, $\alpha S$ der kleinste der beiden Werte ist, außer wenn beide 0,5 sind, wobei dann $\alpha$ 0,5 ist.

2. Sonnenblumenöl gemäß Anspruch 1, **dadurch gekennzeichnet, dass** der Verteilungskoeffizient $\alpha$ der gesättigten Fettsäuren zwischen den Positionen sn-1 und sn-3 wenigstens 0,32 beträgt.

3. Sonnenblumenöl gemäß Anspruch 1 oder 2, **dadurch gekennzeichnet, dass** der Verteilungskoeffizient $\alpha$ der gesättigten Fettsäuren zwischen den Positionen sn-1 und sn-3 wenigsten 0,36 beträgt.

4. Sonneblumenöl gemäß einem der Ansprüche 1 bis 3, **dadurch gekennzeichnet, dass** der Stearinsäuregehalt, bezogen auf den gesamten Fettsäuregehalt, wenigstens 20 % beträgt.

5. Sonnenblumenpflanze, die Samen mit einem Gehalt an einem endogenen Öl gemäß einem der Ansprüche 1 bis 4 bildet.

6. Sonnenblumensamen, hergestellt durch Sonnenblumenpflanzen gemäß Anspruch 5.

7. Eine hybride Sonnenblumenpflanze mit Samen, die ein endogenes Öl mit einem Stearinsäuregehalt von 12 % bis 40,8 %, bezogen auf den gesamten Fettsäuregehalt, aufweisen, und wobei in dem Öl der Ölsäuregehalt größer ist als der Linolsäuregehalt und ein Verteilungskoeffizient $\alpha$ der gesättigten Fettsäuren zwischen den Positionen sn-1 und sn-3 wenigstens 0,28 beträgt, wobei die Pflanze erhältlich ist durch Kreuzung einer ersten Stammpflanze gemäß Anspruch 5 und einer zweiten Stammpflanze mit gewünschten Eigenschaften.

8. Hybride Sonnenblumenpflanze gemäß Anspruch 7, erhältlich durch Kreuzung einer ersten Stammpflanze, die eine Pflanze gemäß Anspruch 5 ist, und einer zweiten Stammpflanze, die eine Pflanze gemäß Anspruch 5 mit anderen gewünschten Eigenschaften ist.

9. Nachkommen der Sonnenblumenpflanze gemäß einem der Ansprüche 5 und 7 bis 8, deren Samen ein endogenes Öl mit einem Stearinsäuregehalt von 12 % bis 40,8 %, bezogen auf den gesamten Fettsäuregehalt, aufweisen, und wobei in dem Öl der Ölsäuregehalt größer ist als der Linolsäuregehalt und ein Verteilungskoeffizient $\alpha$ der gesättigten Fettsäuren zwischen den Positionen sn-1 und sn-3 wenigstens 0,28 beträgt.

10. Sonnenblumenöl gemäß einem der Ansprüche 1 bis 4 zur Herstellung eines Nahrungsproduktes.

11. Nahrungsprodukt, umfassend ein Sonnenblumenöl gemäß einem der Ansprüche 1 bis 4.

**Revendications**

1. Huile de tournesol obtenue directement à partir de graines de tournesol, ayant une teneur en acide stéarique de 12 à 40,8 % par rapport à la teneur totale en acides gras, et dans laquelle la teneur en acide oléique est supérieure à la teneur en acide linoléique de l'huile, **caractérisée en ce que** le coefficient de distribution d'acides gras saturés $\alpha$ entre les positions *sn*-1 et *sn*-3 est d'au moins 0,28, lesquelles graines pouvant être obtenues en faisant pousser des plants de tournesol à partir de graines de tournesol d'une lignée à forte teneur en acide oléique et forte teneur en acide stéarique, en faisant pousser des plants de tournesol à partir des graines de tournesol de la lignée CAS-36, déposée à l'ATCC sous le numéro d'accès PTA-5041, en croisant les lignées pour produire des graines F1 et en récoltant les graines, en produisant des graines F2 et en sélectionnant les graines ayant un indice $\alpha$ supérieur à 0,28, une forte teneur en acide stéarique et une teneur en acide oléique supérieure à la teneur en acide linoléique, dans laquelle le coefficient $\alpha$ est calculé d'après la formule :

$$\alpha S = Min\left[\frac{S_1}{S_1 + S_3}; \frac{S_3}{S_1 + S_3}\right]$$

$S_1$ étant la teneur en acides gras saturés en position TAG *sn-1*, $S_3$ étant la teneur en acides gras saturés en position TAG *sn*-3 et $\alpha$S étant la plus petite des deux valeurs, sauf quand les deux valent 0,5, auquel cas $\alpha$ vaut 0,5.

2. Huile de tournesol selon la revendication 1, **caractérisée en ce que** le coefficient de distribution des acides gras saturés $\alpha$ entre les positions *sn*-1 et *sn*-3 est d'au moins 0,32.

3. Huile de tournesol selon la revendication 1 ou 2, **caractérisée en ce que** le coefficient de distribution des acides gras saturés a entre les positions *sn*-1 et *sn*-3 est d'au moins 0,36.

4. Huile de tournesol selon l'une quelconque des revendications 1 à 3, **caractérisée en ce que** la teneur en acide stéarique, par rapport à la teneur totale en acides gras, est d'au moins 20%.

5. Plant de tournesol qui forme des graines contenant une huile endogène selon les revendications 1 à 4.

6. Graines de tournesol produites par des plants de tournesol selon la revendication 5.

7. Plant de tournesol hybride dont les graines contiennent une huile endogène ayant une teneur en acide stéarique de 12 à 40,8 % par rapport à la teneur totale en acides gras, huile dans laquelle la teneur en acide oléique est supérieure à la teneur en acide linoléique et le coefficient de distribution des acides gras saturés $\alpha$ entre les positions *sn*-1 et *sn*-3 est d'au moins 0,28, lequel plant peut être obtenu par croisement d'un premier plant parent tel que revendiqué dans la revendication 5 et d'un deuxième plant parent ayant des caractéristiques souhaitables.

8. Plant de tournesol hybride selon la revendication 7, pouvant être obtenu par croisement d'un premier plant parent qui est un plant tel que revendiqué dans la revendication 5 et d'un deuxième plant parent qui est un plant tel que revendiqué dans la revendication 5 ayant d'autres caractéristiques souhaitables.

9. Descendance du plant de tournesol de l'une quelconque des revendications 5, 7 et 8, dont les graines contiennent une huile endogène ayant une teneur en acide stéarique de 12 à 40,8 % par rapport à la teneur totale en acides gras, huile dans laquelle la teneur en acide oléique est supérieure à la teneur en acide linoléique et le coefficient de distribution des acides gras saturés $\alpha$ entre les positions sn-1 et sn-3 est d'au moins 0,28.

10. Huile de tournesol selon l'une quelconque des revendications 1 à 4, pour la production d'un produit alimentaire.

11. Produit alimentaire comprenant une huile de tournesol selon l'une quelconque des revendications 1 à 4.

EP 1 689 222 B1

# Fig. 1

```
GPAT
Glycerol-3-phosphate
acyltransferase
```

```
LPAAT
Lysophosphatidic
acyltransferase
```

```
Phosphatidate
phosphatase
```

```
DAGAT
Diacylglycerol
acyltransferase
```

Glycerol-3-P

Lyso-Phosphatidic acid

Phosphatidic acid

Pi

Diglyceride

Triglyceride

# Fig. 2

# Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6475548 B
- WO 9520313 A
- US 4438149 A
- WO 0074470 A

### Non-patent literature cited in the description

- **Álvarez-Ortega. R. ; Cantisán, S. ; Martínez-Force, E. ; Garcés, R.** Characterization of polar and non-polar seed lipid classes from highly saturated fatty acid sunflower mutants. *Lipids,* 1997, vol. 32, 833-837
- **Chow, C. K.** Fatty acids in foods and their health implications. Marcel Dekker, 1992
- **Fernández-Moya, V. ; Martínez-Force, E. ; Garcés, R.** Identification of triacylglycerol species from high-saturated sunflower (Helianthus annuus) mutants. *Agric. Food Chem.,* 2000, vol. 48, 764-769
- **Garcés, R. ; Mancha, M.** One-step lipid extraction and fatty acid methyl esters preparation from fresh plant tissues. *Anal. Biochem.,* 1993, vol. 211, 139-143
- **Gunstone, F.D. ; Harwood, J.L. ; Padley, F.B.** The Lipid Handbook. Chapman and Hall, 1994
- **Laakso, P. ; Christie, W.W.** Chromatographic resolution of chiral diacylglycerol derivatives: potential in the stereo-specific analysis of triacyl-sn-glycerols. *Lipids,* 1990, vol. 25, 349-353
- **Osorio, J. ; Fernández-Martínez, J. ; Mancha, M. ; Garcés, R.** Mutant sunflowers with high concentration of saturated fatty acids in the oil. *Crop Sci.,* 1995, vol. 35, 739-742
- **Reske, J. ; Siebrecht, J. ; Hazebroed, J.** Triacylglycerol composition and structure in genetically modified sunflower and soybean oils. *J. Am. Oil Chem. Soc.,* 1997, vol. 74, 989-998
- **Takagi, T. ; Ando, Y.** Stereospecific analysis of triacyl-sn-glycerols by chiral HPLC. *Lipids,* 1991, vol. 26, 542-547
- **Vander Wal, R.J.** Calculation of the distribution of the saturated and unsaturated acyl groups in fats, from pancreatic lipase hydrolysis data. *J. Am. Oil Chem. Soc.,* 1960, vol. 37, 18-20
- **Soldatov, K.I.** Chemical mutagenesis in sunflower breeding. Proc. 7th Int. Sunflower Association, 1976, 352-357